(19) Europäisches Patentamt
European Patent Office
Office européen des brevets

(11) Veröffentlichungsnummer : **0 061 082**
**B1**

(12) # EUROPÄISCHE PATENTSCHRIFT

(45) Veröffentlichungstag der Patentschrift : **28.11.84**

(51) Int. Cl.$^3$ : **C 07 D279/16**, C 07 D279/20

(21) Anmeldenummer : **82101974.2**

(22) Anmeldetag : **12.03.82**

(54) **Verfahren zur Herstellung von 2,3-Dihydro-4H-1,4-benzothiazinen.**

(30) Priorität : **24.03.81 DE 3111487**

(43) Veröffentlichungstag der Anmeldung : **29.09.82 Patentblatt 82/39**

(45) Bekanntmachung des Hinweises auf die Patenterteilung : **28.11.84 Patentblatt 84/48**

(84) Benannte Vertragsstaaten : **CH DE FR GB IT LI**

(56) Entgegenhaltungen : **MONATSHEFTE FUR CHEMIE, Band 88, Heft 5, 1957, Seiten 822-829, Springer-Verlag**

(73) Patentinhaber : **BAYER AG**
**Konzernverwaltung RP Patentabteilung**
**D-5090 Leverkusen 1 Bayerwerk (DE)**

(72) Erfinder : **Uhrhan, Paul, Dr.**
**Brunnenweg 27**
**D-5068 Odenthal (DE)**
Erfinder : **Krauthausen, Edmund, Dr.**
**Franz-Grillparzer-Ring 4**
**D-5000 Köln 71 (DE)**

**EP 0 061 082 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von 2,3-Dihydro-4H-1,4-benzothiazinen.

Es ist bereits bekannt, 2,3-Dihydro-4H-1,4-benzothiazin durch Umsetzung von 2-Aminothiophenol mit 1,2-Dibromethan in Gegenwart von Kaliumhydroxid herzustellen. Die Ausbeute ist jedoch gering (N.A. Langlet, Bihang. till Svenska Vet.-Akad. Handlinger *22 II*, No. 1, S. 8 ; s. a. Beilstein *XXII*, 4. Aufl., S. 34 (1937)). Als Hauptprodukt entsteht überwiegend Ethylen-bis-2-aminophenylsulfid (s. O. Hromatka, M. Vaculny, J. Augl und K. Wiltschke, Mh. Chem. *88*, 822, 1957 ; E. Fromm, H. Benzinger und F. Schäfer, Ann. Chem. *394*, 332, 1912).

Es ist weiterhin bekannt, 2,3-Dihydro-4H-1,4-benzothiazine durch Reduktion entsprechender 3-Oxo-dihydro-1,4-benzothiazine mit Lithiumaluminiumhydrid herzustellen (s. z. B. O. Hromatkau. Mitarbeiter, Mh. Chem. *88*, 822, 1957 ; J.F. Kerwin, J.E. McCarty u. C.A. Vander, J. Org. Chem. *24*, 1719, 1959), jedoch ist Lithiumaluminiumhydrid nicht ungefährlich in der Handhabung und erfordert den Ausschluß von Feuchtigkeit. Zudem sind mit der Methode nur 3-unsubstituierte Benzothiazinderivate zugänglich.

Nach Culvenor u. Mitarbeitern sollen Dihydrobenzothiazine durch Reaktion von 2-Aminothiophenol mit Oxiranen in alkoholischer Kalilauge gut zugänglich sein (C.C.L. Culvenor, W. Davies und N.S. Heath, J. Chem. Soc. (London) *1949*, 278). Auch bei der Reduktion von 2-Hydroxy-ethyl-2-nitrophenylsulfid mit Zinn und Salzsäure soll 2,3-Dihydro-4H-1,4-benzothiazin entstanden sein (R. Fusco und G. Palazzo, Gazz. chim. ital. *81*, 735, 1951). Später wurde jedoch nachgewiesen, daß die Autoren in beiden Fällen nur die entsprechenden 2-Hydroxyalkylthioaniline in Händen hatten (O. Hromatka, M. Vaculny, J. Augl und K. Wiltschke, Mh. Chem *88*, 822, 1957 ; O. Hromatka, J. Augl, A. Brazda und W. Grünsteidl, Mh. Chem. *90*, 544, 1959).

In der französischen Patentschrift 1 344 437 sind 2,3-Dihydro-4H-1,4-benzothiazine als Ozonschutzmittel für Elastomere beschrieben. Als Herstellungsmöglichkeiten wurde die Methode von Culvenor zitiert, die jedoch nicht zu Benzothiazinen führt, und die Umsetzung von 2-Aminothiophenolen mit 1,2-Dihalogenalkanen oder Chlorhydrinen genannt, ohne jedoch Ausbeuteangaben oder Synthesebeispiele anzuführen.

Es wurde nun gefunden, daß man 2,3-Dihydro-4H-1,4-benzothiazine herstellen kann, wenn man 2-(2-Hydroxyalkyl)-thioaniline mit Hilfe von phosphoriger Säure unter Wasserabspaltung cyclisiert.

Geeignete Konzentrationen an phosphoriger Säure sind 0,1 bis 100, vorzugsweise 5 bis 25 Gew.-%, bezogen auf das eingesetzte 2-(2-Hydroxyalkyl)-thioanilin.

Gegenstand der Erfindung ist daher ein Verfahren zur Überstellung von 2,3-Dihydro-4H-1,4-benzothiazinen, das dadurch gekennzeichnet ist, daß 2-(2-Hydroxyalkyl)-thioaniline der allgemeinen Formel I

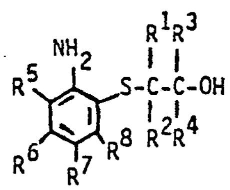

(I)

worin

$R^1$ bis $R^4$ gleich oder verschieden sind und Wasserstoff, niederes Alkyl mit 1 bis 4 C-Atomen, gegebenenfalls substituiertes Cycloalkyl mit 5 bis 8 C-Atomen, Aralkyl mit 7 bis 11 C-Atomen oder Aryl bedeuten, wobei mindestens einer der Reste $R^3$ oder $R^4$ verschieden von Wasserstoff ist, oder

$R^1$ und $R^2$ bzw. $R^3$ und $R^4$ zusammen mit dem C-Atom, an das sie gebunden sind, einen Cycloalkylrest mit 5 bis 8 C-Atomen bilden, oder einer der Reste

$R^3$ oder $R^4$ zusammen mit $R^1$ oder $R^2$ für einen Alkylenrest mit 3 bis 6 C-Atomen steht,

$R^6$ und $R^8$ gleich oder verschieden sind und Wasserstoff, Halogen, Nitro, lineares oder verzweigtes Alkyl mit 1 bis 12 C-Atomen, lineares oder verzweigtes Alkenyl mit 2 bis 12 C-Atomen, Aralkyl mit 7 bis 11 C-Atomen, Cycloalkyl mit 5 bis 8 C-Atomen bedeuten, und

$R^5$ und $R^7$ gleich oder verschieden sind und Wasserstoff, Halogen, Nitro, lineares oder verzweigtes Alkyl mit 1 bis 12 C-Atomen, lineares oder verzweigtes Alkenyl mit 2 bis 12 C-Atomen, Cycloalkyl mit 5 bis 8 C-Atomen, Aralkyl mit 7 bis 11 C-Atomen, Alkoxy, den Rest

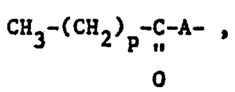

worin

P eine ganze Zahl zwischen 0 und 20, vorzugsweise zwischen 8 und 16 ist, und

A für $—(CH_2)_q—$, $—O—(CH_2)_q—$, $—NH—(CH_2)_q—$ oder $—S—(CH_2)_q—$ mit

$q = 0$ bis 4, vorzugsweise 0 oder 1 steht, oder den Rest

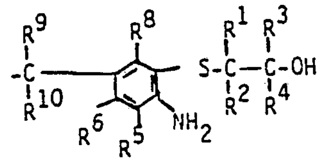

bedeuten,
worin

$R^1$ und $R^6$ und $R^8$ für die oben angegebenen Reste stehen, und

$R^9$ und $R^{10}$ unabhängig voneinander für Wasserstoff, gegebenenfalls substituiertes Aryl, lineares oder verzweigtes Alkyl mit 1 bis 12 C-Atomen, Cycloalkyl oder Cycloalkenyl mit 5 bis 8 C-Atomen stehen, oder

$R^9$ und $R^{10}$ zusammen mit dem C-Atomen, an das sie gebunden sind, einen Cycloalkylrest mit 5 bis 7 C-Atomen bilden mit Hilfe von Phosphoriger Säure unter Wasserabspaltung zu 2,3-Dihydrobenzothiazine der allgemeinen Formel II

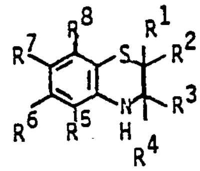

(II)

worin

$R^1$ bis $R^8$ die oben angegebene Bedeutung haben, bei einer Temperatur von 60-300 °C cyclisiert werden. Bevorzugt werden für das erfindungsgemäße Verfahren Verbindungen der Formel I,
worin

$R^1$ und $R^2$ für Wasserstoff oder niederes Alkyl mit 1 bis 4 C-Atomen und
$R^3$ und $R^4$ für niederes Alkyl stehen, und
$R^5$ bis $R^8$ die oben angegebene Bedeutung haben.

Besonders bevorzugt stehen
$R^1$, $R^2$ und $R^5$ bis $R^8$ unabhängig voneinander für Wasserstoff oder Methyl und
$R^3$ und $R^4$ für Methyl.

Als 2-(2-Hydroxyalkyl)-thioaniline seien im einzelnen beispielsweise genannt :

2-(2-Hydroxyethyl)-thioanilin, 2-(2-Hydroxy-propyl-)-thioanilin, 2-(2-Hydroxy-1-methylethyl-) thioanilin, 2-(1,2-Dimethyl-2-Hydroxypropyl)-thioanilin, 2-(2-Hydroxy-1-methylpropyl)-thioanilin, 2-(2-Hydroxy-2-methylpropyl)-thioanilin, 2-(2-Hydroxy-1,1,2-trimethylpropyl)-thioanilin, 2-(2-Hydroxybutyl)-thioanilin, 2-(2-Hydroxypentyl)-thioanilin, 2-(2-Hydroxycyclohexyl)-thioanilin, 2-(2-Hydroxycyclooctyl)-thioanilin, 2-(1,2-Diphenyl-2-hydroxyethyl)-thioanilin, 2-(2-Hydroxy-1-phenylethyl)-thioanilin, 2-(2-Hydroxy-2-phenylethyl)-thioanilin, 2-(2-Hydroxy-2-phenylpropyl-)-thioanilin, 2-(2,2-Diphenyl-2-hydroxyethyl)-thioanilin, 3,5-Dichlor-2-(2-hydroxyethyl)-thioanilin, 3,5-Dichlor-2-(2-hydroxy-propyl)-thioanilin, 3,5-Dichlor-2-(2-hydroxy-2-methylpropyl)-thioanilin, 3,5-Dichlor-2-(2-hydroxy-2-phenylpropyl)-thioanilin, 4-Chlor-2-(2-hydroxy-2-methylpropyl)-thioanilin, 5-Chlor-2-hydroxy-2-methyl-cyclohexyl)-thioanilin, 4-Carboethoxy-2-(2-hydroxy-1,1,2-trimethyl-propyl)-thioanilin, 2-(2-Hydroxyethyl)-thio-4-methoxy-anilin, 4,4'-Diamino-3,3'-di-(2-hydroxy-1,2-dimethylpropyl)-thio-diphenylmethan, 4,4'-Diamino-3,3'-di-(2-hydroxyethyl)-thio-diphenylmethan, 2,2-Di-[4-amino-3-(2-hydroxy-2-methylpropyl)-thio-phenyl]-propan, 4,6-Di-(1-phenylethyl)-2-(2-hydroxy-2-methylpropyl)-thioanilin, 2-(2-Hydroxyethyl)-thio-4-nitro-anilin, 2-(2-Hydroxy-2-methylpropyl)-thio-5-nitroanilin, 2-(2-Hydroxyethylthio-4-stearoyl-anilin, 2-(2-Hydroxy-2-methylpropylthio)-4-hexadecanoyl-anilin, Octadecansäure-4-amino-3-(2-hydroxy-2-phenylpropylthio)-benzylester, 3-Ethoxy-5-brom-6-methyl-2-(2-hydroxy-1-methyl-2-cyclopentyl-3-ethyl-pentylthio)-anilin,

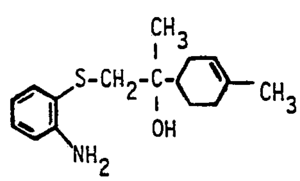

2-[2-Hydroxy-2-(4-methylcyclohex-3-enyl)-propylthio]-anilin,

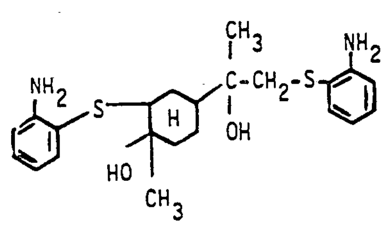

2-[2-Hydroxy-2-(3-0-aminophenylthio-4-hydroxy-4-methylcyclohexyl)-propylthio]-anilin

Die Bildung von 2,3-Dihydro-4H-1,4-benzothiazinen aus 2-(2-hydroxy-alkylthio)-anilinen kann anhand der folgenden Gleichung erläutert werden.

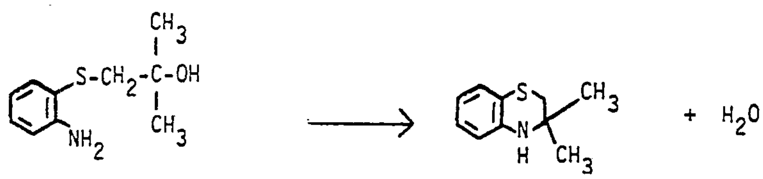

Die Umsetzung kann im Temperaturbereich von 60-300 °C, vorzugsweise von 100-200 °C durchgeführt werden, wobei man bei Normaldruck, erhöhtem oder vermindertem Druck arbeiten kann. Vorzugsweise wird bei Normaldruck gearbeitet. Es hat sich als günstig herausgestellt, das bei der Reaktion entstehende Reaktionswasser aus dem Ansatz zu entfernen. Dies kann z. B. dadurch geschehen, daß man das Reaktionswasser aus dem Ansatz bei erhöhter Temperatur und/oder vermindertem Druck herausdestilliert. Vorzugsweise verwendet man hierzu ein geeignetes organisches Lösungsmittel als Schleppmittel. Geeignete Lösungsmittel sind solche, die zusammen mit Wasser azeotrop sieden. Besonders bevorzugte Lösungsmittel sind mit Wasser nicht mischbar, wie z. B. Toluol, Xylol, Chloroform und Tetrachlorkohlenstoff. In einer besonderen Ausführungsform kann das Destillat in Schleppmittel und Wasser getrennt werden und das Schleppmittel in die Destillation oder Umsetzung zurückgeführt werden.

Die nach dem erfindungsgemäßen Verfahren hergestellten 2,3-Dihydro-4H-1,4-benzothiazine können Verwendung finden als Ozonschutzmittel für Elastomere (vergl. französisches Patent 1 344 437), als Zwischenprodukte für die Herstellung hochwirksamer Stabilisatoren für organische Verbindungen, als Zwischenprodukte für Farbstoffe (vergl. McNally und Dickey, US-Patent 2 251 945), als Zwischenprodukte für die Herstellung von Antimykotika (vergl. Böshagen und Plempel, US-Patent 3 911 126) oder als gut wirkende Alterungsschutzmittel für Polymere, mit dem Vorteil, daß sie das damit stabilisierte Polymere nicht oder nur ganz schwach verfärben.

Die folgenden Beispiele sollen das erfindungsgemäße Verfahren näher erläutern.

## Beispiele

### Beispiel 1

18,25 kg 2-(2-Hydroxy-2-methylpropylthio)-anilin (erhalten nach der Methode von Culvenor u. a., J. Chem. Soc. *1949*, 278 ff, aus 2-Amino-thiophenol, Kaliumhydroxid und Isobutenoxid mit Sdp. 131-9 °C/0,1 mbar, Schmp. 45-49 °C) erhitzt man 2 Std. mit 1,85 kg Xylol und 2,08 kg 73 %iger phosphoriger Säure am Wasserabscheider. Anschließend wird mit 2,775 kg (69,4 Mol) Natriumhydroxid in 12 kg Wasser neutralisiert, die organische Phase abgetrennt und das Lösungsmittel im Vakuum entfernt. Nach Destillation erhält man 10,76 kg 3,3-Dimethyl-2,3-dihydro-4H-1,4-benzothiazin mit Sdp. 110-115 °C/0,4 mbar, Schmp. 52 bis 55 °C. Das Massenspektrum zeigt ein Molekülion bei 179 (64 % rel. Intensität) und als Hauptfragmente 164 (100 %) und 149 (30 %).

### Beispiel 2

Analog Beispiel 1 wurde mit 2-(2-Hydroxy-1,1,2-trimethylpropylthio)-anilin, Schmp. 60-2 °C, Sdp. 125 °C/0,1 mbar (erhalten aus Tetramethyl-oxiran und 2-Aminothiophenol) das gewünschte 2,2,3,3-Tetramethyl-2,3-dihydro-4H-1,4-benzothiazin als farbloses Öl mit Sdp. 89 °C/0,04 mbar erhalten. $n_D^{20}$ 1,595 5 ; das Massenspektrum zeigt ein Molekülion bei 207 (74 % rel. Intensität) und als Hauptfragmente 192 (24 %), 177 (14 %), 164 (100 %), 150 (44 %), 132 (70 %), 125 (24 %), 109 (15 %), 92 (22 %), 42 (17 %) und 41 (16 %).

### Beispiel 3

In eine Lösung aus 125 g (1 Mol) 2-Aminothiophenol, 41 g (1,025 Mol) Natriumhydroxid und 200 ml Wasser tropft man bei 50 °C 90 g (0,5 Mol) Limonendiepoxid. Es bildet sich ein trüber harziger Niederschlag, der abgetrennt, gewaschen und im Vakuum bei 70 °C von anhaftenden flüchtigen Anteilen befreit wird. Man erhält 140 g eines zähen hellgelben Öls, das mit 100 ml Xylol und 14 g 73 %iger phosphoriger Säure versetzt und 6 Std. am Wasserabscheider gekocht wurde. Nach Neutralisieren mit Natronlauge wurde die organische Phase abgetrennt und das Lösungsmittel im Vakuum entfernt. Man erhält 75 g eines braunen spröden Pulvers, das als Hauptmenge 10a-Methyl-3-(3-methyl-2,3-dihydro-4H-1,4-benzothiazin-3-yl)-1,2,3,4,4a,10a-hexahydrophenothiazin enthält. Das Massenspektrum zeigt als Molekülion 387 (12 % rel. Intensität) und als Hauptfragmente 258 (40 %), 218 (30 %), 164 (100 %), 149 (19 %), 136 (17 %), 133 (20 %), 125 (28 %) und 124 (27 %).

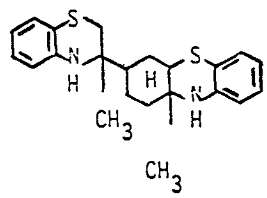

## Beispiel 4

34 g (0,2 Mol) 4- bzw. 5-Nitro-2-thioanilin (Gemisch) und 8,2 g (0,205 Mol) Natriumhydroxid in 150 ml Wasser werden mit 14,4 g (0,2 Mol) Isobutenoxid unter Kühlung versetzt, 3 Std. nachgerührt und abgesaugt. Man erhielt 46 g 2-(2-Hydroxy-2,2-dimethylethyl)-thio-4- bzw. 5-nitroanilin als olivbraunes Pulver mit Schmp. 77-90 °C, dessen Massenspektrum ein Molekülion bei 242 (31 % rel. Intensität) und als Hauptfragmente 184 (59 %), 167 (100 %), 137 (39 %) und 59 (75 %) zeigt. Nach Cyclisierung mit phosphoriger Säure in Xylol, wie in Beispiel 1 beschrieben, erhält man ein nicht destillierbares dunkelbraunes Zähes Öl, das aufgrund der Spektren überwiegend aus 3,3-Dimethyl-6- bzw. 7-nitro-2,3-dihydro-4H-1,4-benzothiazin besteht. Das Massenspektrum zeigt ein Molekülion bei 224 (100 % rel. Intensität) und als Hauptfragmente 209 (87 %), 181 (92 %), 169 (41 %), 163 (34 %) und 55 (76 %).

Beispiele 5-7 zeigen die technische Verwertbarkeit der nach dem erfindungsgemäßen Verfahren hergestellten Verbindungen als Zwischenprodukte für die Herstellung von Stabilisatoren.

## Beispiel 5

Zu 90 g (0,5 Mol) des Produktes aus Beispiel 1 und 10 g Aluminium-trichlorid werden bei 100 °C 112 g (1 Mol) Diisobutylen zugetropft und noch 6 Std. bei dieser Temperatur nachgerührt ; die Reaktionsmischung wird auf Eiswasser gegossen, der Niederschlag abgesaugt und in Natronlauge eingetragen. Die organische Schicht wird abgetrennt, im Hochvakuum von unumgesetztem Edukt befreit und der Rückstand aus Methanol/Wasser umkristallisiert. Man erhält 3,3-Dimethyl-7-(1,1,3,3-tetramethylbutyl)-2,3-dihydro-4H-1,4-benzothiazin mit Schmp. 60-2 °C. Das Massenspektrum zeigt ein Molekülion bei 291 (13 % rel. Intensität) und ein Hauptfragment bei 220 (100 %).

## Beispiel 6

Analog Beispiel 5 erhält man mit α-Methylstyrol anstelle von Diisobutylen 92 g 3,3-Dimethyl-5,7-di-(1-methyl-1-phenylethyl)-2,3-dihydro-4H-1,4-benzothiazin als gelbes Öl. Das Massenspektrum zeigt ein Molekülion bei 415 (91 % rel. Intensität) und als Hauptfragmente 400 (100 %), 322 (11 %), 119 (38 %), 103 (11 %), 91 (31 %).

## Beispiel 7

12 g 2,2,3,3-Tetramethyl-2,3-dihydro-4H-1,4-benzothiazin (Beispiel 2), 25 ml Methanol und 2 Tropfen konz. Schwefelsäure werden vorgelegt und bei 30 °C 2,4 g 37 %ige wäßrige Formaldehydlösung zugetropft. Man kocht 2 Std. unter Rückfluß, neutralisiert mit wäßriger Natronlauge, kocht weitere 3 Std. unter Rückfluß. Anschließend destilliert man das Lösungsmittel weitgehend im Vakuum ab, nimmt in Methylenchlorid auf, und wäscht zweimal mit Wasser. Das Lösungsmittel wird abdestilliert, und man erhält 8,2 g Di-(2,2,3,3-tetramethyl-2,3-dihydro-4H-1,4-benzothiazin-7-yl)-methan als zähes Harz. Das Massenspektrum zeigt ein Molekülion bei 426 (94 % rel. Intensität) und die Hauptfragmente 411 (18 %) und 383 (100 %). Daneben enthält das Produkt noch geringe Mengen drei und vier (2,2,3,3-Tetramethyl-2,3-dihydro-4H-1,4-benzothiazin)-Kerne enthaltende Verbindungen mit den Molmassen 645 und 864.

## Beispiel 8

Beispiel 8 zeigt die technische Verwertbarkeit als Stabilisatoren in Kautschuk.

Folgende Naturkautschuk-Mischung wurde auf der Walze hergestellt :

| | Gew.-Teile |
|---|---|
| Helle Crepe | 100,0 |
| Zinkoxid | 10,0 |
| Stearinsäure | 1,0 |
| Titandioxid | 10,0 |
| Blanc fixe | 60,5 |
| Tetramethylthiuramdisulfid | 0,5 |
| Schwefel | 2,0 |
| Stabilisator | wie in Tabelle 1 angegeben |

Es wurde 15 min. bei 130 °C in der Presse vulkanisiert. Das erhaltene Vulkanisat wurde einer Alterung in der Sauerstoffbombe (nach Bierer-Davis) bei 21 bar Sauerstoff und 70 °C ausgesetzt (DIN 53 508). Die Ergebnisse sind in Tabelle 1 zusammengefaßt und verdeutlichen die Verwendbarkeit der nach dem erfindungsgemäßen Verfahren hergestellten Verbindungen als Stabilisatoren bzw. als Zwischenprodukte für Stabilisatoren.

Tabelle 1

a) ohne Alterungsschutzmittel (Vergleich)

| | Festigkeit (mPa) | Dehnung (%) | Modul bei 450 % Dehnung (mPa) | Elastizität 20 °C/70 °C (%) | | Härte 20 °C/70 °C (Shore A) | |
|---|---|---|---|---|---|---|---|
| Vor der Alterung | 22,7 | 730 | 8,1 | 74 | 79 | 52 | 53 |
| Nach 7 Tg. Alterung | 5,0 | 470 | — | — | 50 | 47 | 35 |
| Nach 14 Tg. Alterung | zerstört | | | | | | |

Farbe der Vulkanisate :

Vor der Belichtung weiß
Nach 2 Monaten Tagesbelichtung weiß

b) Mit 1 Gew.-Teil 2,2,4-Trimethyl-1,2-dihydrochinolin, polymerisiert (Vergleich)

| | Festigkeit | Dehnung | Modul | Elastizität | | Härte | |
|---|---|---|---|---|---|---|---|
| Vor der Alterung | 23,8 | 710 | 9,6 | 75 | 81 | 54 | 53 |
| Nach 7 Tg. Alterung | 16,8 | 580 | 11,5 | 72 | 79 | 56 | 55 |
| Nach 14 Tg. Alterung | 14,4 | 570 | 10,4 | 66 | 75 | 54 | 53 |
| Nach 21 Tg. Alterung | 11,9 | 540 | 9,8 | 55 | 63 | 53 | 50 |

Farbe der Vulkanisate :

Vor der Belichtung rosa
Nach 2 Monaten Tagesbelichtung hellbraun

c) Mit 1 Gew.-Teil alkyl- und aralkyl-substituierter Phenole (Vergleich)

| | | | | | | | |
|---|---|---|---|---|---|---|---|
| Vor der Alterung | 23,0 | 730 | 8,0 | 74 | 81 | 52 | 52 |
| Nach 7 Tg. Alterung | 14,6 | 600 | 8,9 | 65 | 69 | 52 | 50 |
| Nach 14 Tg. Alterung | 9,3 | 560 | 7,1 | 57 | 56 | 46 | 38 |
| Nach 21 Tg. Alterung | zerstört | | | | | | |

Farbe der Vulkanisate :

Vor der Belichtung weiß
Nach 2 Monaten Tagesbelichtung weiß

d) Mit 1 Gew.-Teil 2,2'-Methylen-bis-(4-methyl-6-tert.-butyl-phenol) (Vergleich)

| | Festigkeit | Dehnung | Modul | Elastizität | | Härte | |
|---|---|---|---|---|---|---|---|
| Vor der Alterung | 22,8 | 750 | 7,5 | 74 | 79 | 51 | 51 |
| Nach 7 Tg. Alterung | 17,8 | 650 | 9,6 | 63 | 77 | 52 | 51 |
| Nach 14 Tg. Alterung | 15,4 | 600 | 9,7 | 62 | 67 | 49 | 48 |
| Nach 21 Tg. Alterung | 13,2 | 610 | 8,8 | 57 | 65 | 47 | 45 |

Tabelle 1 (Fortsetzung)

Farbe der Vulkanisate :

| | |
|---|---|
| Vor der Belichtung | weiß |
| Nach 2 Monaten Tagesbelichtung | rosa |

e) Mit 1 Gew.-Teil Stabilisator aus Beispiel 1

| | | | | | | | |
|---|---|---|---|---|---|---|---|
| Vor der Alterung | 21,4 | 750 | 5,7 | 73 | 79 | 49 | 48 |
| Nach 14 Tg. Alterung | 12,2 | 630 | 6,6 | 57 | 66 | 46 | 47 |
| Nach 21 Tg. Alterung | 8,8 | 600 | 5,4 | 53 | 63 | 45 | 45 |

Farbe der Vulkanisate :

| | |
|---|---|
| Vor der Belichtung | weiß |
| Nach 2 Monaten Tagesbelichtung | creme |

f) Mit 1 Gew.-Teil Stabilisator aus Beispiel 5

| | | | | | | | |
|---|---|---|---|---|---|---|---|
| Vor der Alterung | 23,6 | 740 | 8,4 | 74 | 81 | 52 | 52 |
| Nach 7 Tg. Alterung | 19,4 | 620 | 11,2 | 70 | 76 | 55 | 55 |
| Nach 14 Tg. Alterung | 14,2 | 550 | 10,9 | 64 | 73 | 53 | 52 |
| Nach 21 Tg. Alterung | 11,5 | 530 | 9,8 | 58 | 69 | 51 | 48 |

Farbe der Vulkanisate :

| | |
|---|---|
| Vor der Belichtung | weiß |
| Nach 2 Monaten Tagesbelichtung | schwach creme |

g) Mit 1 Gew.-Teil Stabilisator aus Beispiel 6

| | Festigkeit | Dehnung | Modul | Elastizität | | Härte | |
|---|---|---|---|---|---|---|---|
| Vor der Alterung | 22,9 | 730 | 8,7 | 73 | 79 | 49 | 50 |
| Nach 7 Tg. Alterung | 14,1 | 620 | 8,9 | 60 | 70 | 51 | 51 |
| Nach 14 Tg. Alterung | 9,4 | 580 | 6,9 | 49 | 56 | 46 | 39 |
| Nach 21 Tg. Alterung | zerstört | | | | | | |

Farbe der Vulkanisate :

| | |
|---|---|
| Vor der Belichtung | weiß |
| Nach 2 Monaten Tagesbelichtung | schwach creme |

**Ansprüche**

1. Verfahren zur Herstellung von 2,3-Dihydro-4H-1,4-benzothiazinen, dadurch gekennzeichnet, daß man 2-(2-Hydroxyalkyl)-thioaniline der allgemeinen Formel I

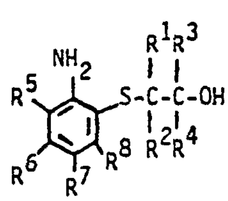

(I)

worin

$R^1$ bis $R^4$ gleich oder verschieden sind und Wasserstoff, niederes Alkyl mit 1-4 C-Atomen, Cycloalkyl mit 5-8 C-Atomen, Aralkyl mit 7-11 C-Atomen oder Aryl bedeuten, wobei mindestens einer der Reste $R^3$ oder $R^4$ verschieden von Wasserstoff ist, oder

$R^1$ und $R^2$ bzw. $R^3$ und $R^4$ zusammen mit dem C-Atomen, an das sie gebunden sind, einen Cycloalkylrestmit 5-8 C-Atomen bilden, oder einer der Reste

$R^3$ oder $R^4$ zusammen mit $R^1$ oder $R^2$ für einen Alkylenrest mit 3-6 C-Atomen steht,

$R^6$ und $R^8$ gleich oder verschieden sind und Wasserstoff, Halogen, Nitro, lineares oder verzweigtes Alkyl mit 1-12 C-Atomen, lineares oder verzweigtes Alkenyl mit 2-12 C-Atomen, Aralkyl mit 7-11 C-Atomen, Cycloalkyl mit 5 bis 8 C-Atomen oder Alkoxy bedeuten, und

$R^5$ und $R^7$ gleich oder verschieden sind und Wasserstoff, Halogen, Nitro, lineares oder verzweigtes Alkyl mit 1-12 C-Atomen, lineares oder verzweigtes Alkenyl mit 2-12 C-Atomen, Cycloalkyl mit 5-8 C-Atomen, Aralkyl mit 7-11 C-Atomen, Alkoxy, den Rest

$$CH_3-(CH_2)_p-\underset{\underset{O}{\|}}{C}-A- ,$$

worin

p eine ganze Zahl zwischen 0 und 20, vorzugsweise zwischen 8 und 16 ist, und

A für $-(CH_2)_q-$, $-O-(CH_2)_q-$, $-NH-(CH_2)_q-$ oder $-S-(CH_2)_q-$ mit

q = 0 bis 4, vorzugsweise 0 oder 1 steht, oder den Rest

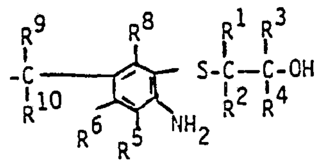

bedeuten,

worin

$R^1$ bis $R^6$ und $R^8$ die oben angegebene Bedeutung haben, und

$R^9$ und $R^{10}$ unabhängig voneinander für Wasserstoff, gegebenenfalls substituiertes Aryl, lineares oder verzweigtes Alkyl mit 1 bis 12 C-Atomen, Cycloalkyl oder Cycloalkenyl mit 5-8 C-Atomen stehen, oder

$R^9$ und $R^{10}$ zusammen mit dem C-Atom, an das sie gebunden sind, einen Cycloalkylrest mit 5-7 C-Atomen bilden, mit Hilfe von phosphoriger Säure unter Wasserabspaltung zu 2,3-Dihydro-4H-1,4-benzothiazinen der allgemeinen Formel II

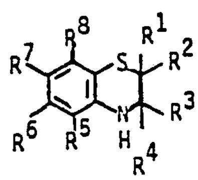

(II)

worin

$R^1$ bis $R^8$ die oben angegebene Bedeutung haben, mit Hilfe von phosphoriger Säure bei einer Temperatur von 60-300 °C unter Wasserabspaltung, cyclisiert.

2. Verfahren nach Anspruch 1, dadurch gekennzeichnet, daß die Wasserabspaltung bei 100 bis 200 °C durchgeführt wird.

3. Verfahren nach Anspruch 1, dadurch gekennzeichnet, daß man das bei der Reaktion entstehende Wasser aus dem Reaktionsansatz entfernt.

4. Verfahren nach Anspruch 1, dadurch gekennzeichnet, daß man die Wasserabspaltung in Gegenwart von 0,1 bis 100 Gew.-% phosphoriger Säure durchführt.

5. Verfahren nach Anspruch 1, dadurch gekennzeichnet, daß man die Wasserabspaltung in Gegenwart von 5-25 Gew.-% phosphoriger Säure durchführt.

**Claims**

1. Process for the preparation of 2,3-dihydro-4H-1,4-benzothiazines, characterised in that 2-(2-hydroxyalkyl)-thioanilines of the general formula I

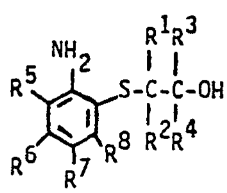

(I)

wherein

$R^1$ to $R^4$ are identical or different and denote hydrogen, lower alkyl with 1-4 C atoms, cycloalkyl with 5-8 C atoms, aralkyl with 7-11 C atoms or aryl, at least one of the radicals $R^3$ or $R^4$ being different from hydrogen, or

$R^1$ and $R^2$ or $R^3$ and $R^4$, together with the C atom to which they are bonded, form a cycloalkyl radical with 5-8 C atoms, or one of the radicals

$R^3$ or $R^4$, together with $R^1$ or $R^2$, represents an alkylene radical with 3-6 C atoms,

$R^6$ and $R^8$ are identical or different and denote hydrogen, halogen, nitro, linear or branched alkyl with 1-12 C atoms, linear or branched alkenyl with 2 to 12 C atoms, aralkyl with 7-11 C atoms, cycloalkyl with 5 to 8 C atoms or alkoxy, and

$R^5$ and $R^7$ are identical or different and denote hydrogen, halogen, nitro, linear or branched alkyl with 1-12 C atoms, linear or branched alkenyl with 2-12 C atoms, cycloalkyl with 5-8 C atoms, aralkyl with 7-11 C atoms, alkoxy, the radical

$$CH_3-(CH_2)_p-\overset{\overset{\displaystyle O}{\|}}{C}-A- ,$$

wherein

p is an integer between 0 and 20, preferably between 8 and 16, and

A represents $-(CH_2)_q-$, $-O-(CH_2)_q-$, $-NH-(CH_2)_q-$ or $-S-(CH_2)_q-$ in which q represents 0 to 4, preferably 0 or 1, or the radical

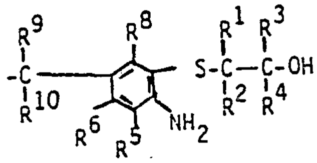

wherein

$R^1$ to $R^6$ and $R^8$ have the meaning given above, and $R^9$ and $R^{10}$ independently of one another represent hydrogen, optionally substituted aryl, linear or branched alkyl with 1 to 12 C atoms, cycloalkyl or cycloalkenyl with 5-8 C atoms, or

$R^9$ and $R^{10}$, together with the C atom to which they are bonded, form a cycloalkyl radical with 5-7 C atoms, are cyclised with the aid of phosphorous acid, with the elimination of water, to form 2,3-dihydro-4H-1,4-benzothiazines of the general formula II

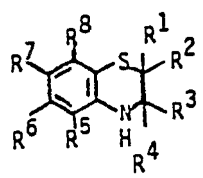

(II)

wherein

$R^1$ to $R^8$ have the meaning given above, with the aid of phosphorous acid at a temperature of 60-300 °C, with the elimination of water.

2. Process according to Claim 1, characterised in that the elimination of water is carried out at 100 to 200 °C.

3. Process according to Claim 1, characterised in that the water forming during the reaction is removed from the reaction mixture.

4. Process according to Claim 1, characterised in that the elimination of water is carried out in the presence of 0.1 to 100 % by weight of phosphorous acid.

5. Process according to Claim 1, characterised in that the elimination of water is carried out in the presence of 5-25 % by weight of phosphorous acid.

## Revendications

1. Procédé de production de 2,3-dihydro-4H-1,4-benzothiazines, caractérisé en ce qu'on cyclise des 2-(2-hydroxyalkyl)-thioanilines de formule générale I

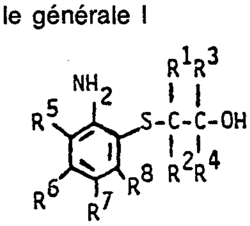

(I)

dans laquelle

$R^1$ à $R^4$ sont égaux ou différents et représentent l'hydrogène, un groupe alkyle inférieur ayant 1 à 4 atomes de carbone, un groupe cycloalkyle ayant 5 à 8 atomes de carbone, un groupe aralkyle ayant 7 à 11 atomes de carbone ou un groupe aryle, l'un au moins des restes $R^3$ ou $R^4$ étant différent de l'hydrogène, ou

$R^1$ et $R^2$ et respectivement $R^3$ et $R^4$ forment conjointement avec l'atome de carbone auquel ils sont liés un reste cycloalkyle ayant 5 à 8 atomes de carbone, ou bien l'un des restes

$R^3$ ou $R^4$ conjointement avec $R^1$ ou $R^2$ forment un reste alkylène ayant 3 à 6 atomes de carbone,

$R^6$ et $R^8$ sont égaux ou différents et représentent l'hydrogène, un halogène, un groupe nitro, un groupe alkyle linéaire ou ramifié ayant 1 à 12 atomes de carbone, un groupe alcényle linéaire ou ramifié ayant 2 à 12 atomes de carbone, un groupe aralkyle ayant 7 à 11 atomes de carbone, un groupe cycloalkyle ayant 5 à 8 atomes de carbone ou un groupe alkoxy, et

$R^5$ et $R^7$ sont égaux ou différents et représentent l'hydrogène, un halogène, un groupe nitro, un groupe alkyle linéaire ou ramifié ayant 1 à 12 atomes de carbone, un groupe alcényle linéaire ou ramifié ayant 2 à 12 atomes de carbone, un groupe cycloalkyle ayant 5 à 8 atomes de carbone, un groupe aralkyle ayant 7 à 11 atomes de carbone, un groupe alkoxy, le reste

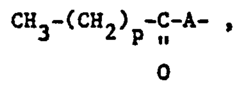

dans lequel

p représente un nombre entier compris entre 0 et 20, de préférence entre 8 et 16 et

A est un groupe $-(CH_2)_q-$, $-O-(CH_2)_q-$, $-NH-(CH_2)_q-$ ou $-S-(CH_2)_q-$, avec q = 0 à 4, de préférence 0 ou 1, ou le reste

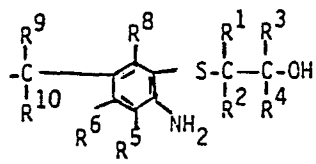

où

$R^1$ à $R^6$ et $R^8$ ont la définition indiquée ci-dessus et

$R^9$ et $R^{10}$ représentent, indépendamment l'un de l'autre, l'hydrogène, un groupe aryle éventuellement substitué, un groupe alkyle linéaire ou ramifié ayant 1 à 12 atomes de carbone, un groupe cycloalkyle ou cycloalcényle ayant 5 à 8 atomes de carbone, ou bien

$R^9$ et $R^{10}$ forment conjointement avec l'atome de carbone auquel ils sont liés un reste cycloalkyle ayant 5 à 7 atomes de carbone, à l'aide d'acide phosphoreux avec élimination d'eau, en 2,3-dihydro-4H-1,4-benzothiazines de formule générale II

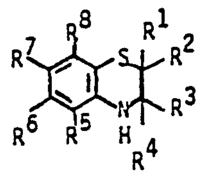

(II)

dans laquelle

$R^1$ à $R^8$ ont la définition indiquée ci-dessus, au moyen d'acide phosphoreux à une température de 60 à 300 °C, avec élimination d'eau.

2. Procédé suivant la revendication 1, caractérisé en ce que l'élimination d'eau est effectuée à une température de 100 à 200 °C.

3. Procédé suivant la revendication 1, caractérisé en ce qu'on élimine du mélange réactionnel l'eau formée au cours de la réaction.

4. Procédé suivant la revendication 1, caractérisé en ce qu'on effectue l'élimination de l'eau en présence de 0,1 à 100 % en poids d'acide phosphoreux.

5. Procédé suivant la revendication 1, caractérisé en ce qu'on effectue l'élimination de l'eau en présence de 5 à 25 % en poids d'acide phosphoreux.